# EUROPÄISCHE PATENTSCHRIFT

(11) **EP 0 578 344 B1**
(45) Veröffentlichungstag und Bekanntmachung des Hinweises auf die Patenterteilung: **28.08.1996**
(21) Anmeldenummer: 93250170.3
(22) Anmeldetag: 15.06.1993
(51) Int. Cl.: C07C 279/26, A61K 7/22

(54) **Modifiziertes Chlorhexidin-Addukt**
Modified chlorhexidine adduct
Produit d'addition modifié de la chlorhexidine

(30) Priorität: 08.07.1992 DE 4222821
(43) Veröffentlichungstag der Anmeldung: 12.01.1994
(73) Patentinhaber: IVOCLAR AG, FL-9494 Schaan (LI)
(72) Erfinder: Rheinberger, Volker, Dr., FL-9490 Vaduz (LI); Burtscher, Peter, Dr., A-6714 Nütziders (AT); Salz, Ulrich, Dr., D-8995 Weissenberg (DE)
(74) Vertreter: UEXKÜLL & STOLBERG

(56) Entgegenhaltungen:
- DE-A- 2 158 150
- CHEMICAL ABSTRACTS, vol. 105, 1986, Columbus, Ohio, US; abstract no. 146197c,
- CHEMICAL ABSTRACTS, vol. 95, 1981, Columbus, Ohio, US; abstract no. 103346h,

## Beschreibung

Die Erfindung betrifft ein Chlorhexidin-Addukt, das als Antiseptikum und insbesondere als Antiseptikum in der Zahnheilkunde sowie als therapeutisches und prophylaktisches Antiplaquemittel eingesetzt werden kann.

Bei dem Versuch, die Bildung von Plaque und damit auch von Karies zu inhibieren bzw. vollständig zu unterbinden, sind in der Vergangenheit Substanzen mit antibakteriellen Eigenschaften wie z.B. chlorierte Phenole, Formaldehyd und quartäre Ammoniumverbindungen auf ihre Wirksamkeit hin überprüft worden. Diese haben aufgrund ihrer Toxizität und ihres eingeschränkten Wirkungsspektrums jedoch keinen Eingang in der Praxis gefunden.

Das derzeit wirksamste Antiplaquemittel ist Chlorhexidin (1,6-bis-(N⁵-p-Chlorphenyl-N'-diguanido)-hexan), welches insbesondere in Form des wasserlösliches Digluconats, aber auch als schwerlösliches Diacetat und Dihydrochlorid verwendet wird (vgl. hierzu, A. Scheie in J. Dent. Res. 68, 1609 (1989) und P. Gjermo in J. Dent. Res. 68, 1602 (1989)).

Neben diesen Chlorhexidinverbindungen ist auch Chlorhexidin-Dihydrofluorid bekannt, welches gemäß DE-OS 21 58 150 als antiseptisches Mittel in transparenten Zahngelen Verwendung findet.

Ferner ist aus I. Ostela und J. Tenovuo in Scand. J. Dent. Res. 98, 1 (1990) eine Mischung aus Chlorhexidin, Aminfluorid und Zinndifluorid bekannt. Diese Mischung kann in Zahngelen als Bakterizid gegenüber kariogenen Bakterien eingesetzt werden.

Es ist gezeigt worden, daß mit der Verwendung von Chlorhexidin als Chemotherapeutikum Bakterien vom Typ Streptococcus mutans wirksam begegnet werden kann. Bakterien dieses Typs spielen bei der Bildung von Karies an menschlichen Zähnen eine wesentliche Rolle. Daher wird angenommen, daß mit der Verringerung ihrer Menge an Zahnoberflächen der Bildung von Karies wirksam entgegengetreten werden kann (vgl. hierzu I. Ostela und J. Tenovuo in Scand. J. Dent. Res. 98, 1 (1990)).

Die bakterizide Wirkung, die Chlorhexidin gegenüber Bakterien des Typs Streptococcus mutans ausübt, ist jedoch stark abgeschwächt, wennn es in geringen Konzentrationen eingesetzt wird. Daher ist auch Chlorhexidin in der praktischen Anwendung deutlichen Einschränkungen unterworfen, wenn es darum geht, Zahnplaque zu verringern, die ihrerseits zur Entstehung von Parodontose und Karies führen kann. Darüber hinaus kann die Anwendung von Chlorhexidin in höheren Konzentrationen zu unerwünschten Verfärbungen der Zunge, Zähne, Prothesen und Füllungen führen (vgl. hierzu L. Flötra, P. Gjermo, G. Rölla und J. Waerhaug in Scand. J. Dent. Res. 79, 119 (1971)).

Zinn-Ionen zeigen eine signifikante Antikarieswirkung und einen plaqueinhibierenden Effekt. Zum einen wird der Metabolismus der in der Plaque vorhandenen Mikroorganismen gestört (siehe z.B. J.E. Ellingsen, B. Svatun und G. Rölla, Acta Odontol. Scand. 38, 219 (1980) und N. Tinanoff, J.M. Brady und A. Gross, Caries Res. 10, 415 (1976)), zum anderen lagern sich Zinn(II)-Ionen auf der Zahnoberfläche ab und bilden dort säureresistente Präzipitate zusammen mit Fluorid-, Calcium- und Phosphationen (siehe z.B. J.E. Ellingsen, Scand. J. Dent. Res. 94, 299 (1986) und J.E. Ellingsen und G. Rölla, Scand. J. Dent. Res. 95, 281 (1987)).

Der Erfindung liegt daher die Aufgabe zugrunde, ein Chlorhexidin-Addukt zur Verfügung zu stellen, das als Antiplaquemittel selbst in sehr geringen Konzentrationen wirksam der Neubildung und dem Wachstum von Zahnbelägen entgegenwirkt, empfindliche Zahnhälse desensibilisieren kann und darüber hinaus durch Abgabe von Fluorid in der Lage ist, den Zahnschmelz vor einer Demineralisierung insbesondere durch Säuren zu schützen.

Diese Aufgabe wird überraschenderweise durch das Chlorhexidin-Addukt gemäß Anspruch 1 und das Verfahren zu seiner Herstellung gemäß den Ansprüchen 2, 3 und 4 sowie seine Verwendung gemäß den Ansprüchen 5 und 6 gelöst.

Bei dem erfindungsgemäßen Chlorhexidin-Addukt handelt es sich um eine Verbindung mit folgender Formel: 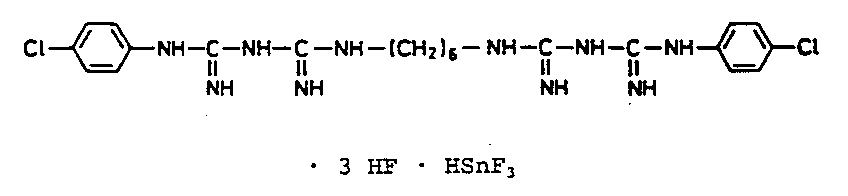
oder dessen Hydrate.

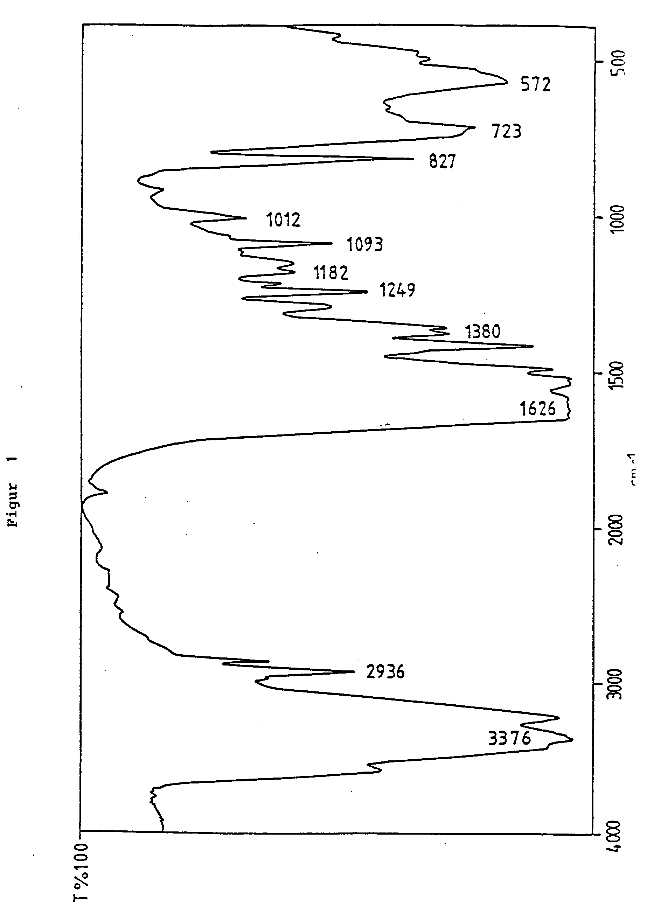

Das Addukt weist das IR-Spektrum gemäß Figur 1 auf. In welcher Weise das erfindungsgemäße Addukt aufgebaut ist, ist nicht bekannt. Grundsätzlich ist vorstellbar, daß das erfindungsgemäße Addukt aus elektrisch neutralen Molekülen besteht oder in Form von Ionen und damit als Salz vorliegt.

Das erfindungsgemäße Addukt wird hergestellt, indem ein Chlorhexidinsalz (bevorzugt Chlorhexidindigluconat), Zinndifluorid und Fluorwasserstoff in einem Molverhältnis von 1 : 1 bis 4 : 4 bis 8 in einem Gemisch aus 3:1 Vol.-Teilen Ethanol/Wasser als Lösungsmittel umgesetzt werden und der entstandene Niederschlag abgetrennt wird.

Bevorzugt wird das erfindungsgemäße Chlorhexidin-Addukt dadurch hergestellt, daß die Reaktion bei Raumtemperatur durchgeführt wird und das Molverhältnis von Chlorhexidinsalz zu Zinndifluorid zu Fluorvasserstoff 1 : 4 : 6 beträgt. Die hierbei erzielbaren Ausbeuten betragen 90 bis annähernd 100%.

Bei der Durchführung des Herstellungsverfahrens sind erhöhte Temperaturen nachteilig, da sie die Bildung von Mischprodukten mit geringerem Gehalt an Zinnfluorid fördern.

Als Reaktionsdauer sind typischerweise 24 Stunden ausreichend, um zu einer vollständigen Reaktion zu kommen. Abhängig von den gewählten Reaktionsparametern kann die Dauer der Reaktion jedoch variieren. Die für den jeweiligen Fall am besten geeignete Reaktionsdauer kann jedoch in einfacher Weise durch Routineexperimente ermittelt werden.

Das bei der Reaktion überwiegend als Niederschlag anfallende Chlorhexidin-Addukt wird bevorzugt durch Filtration und anschließendes Waschen mit Wasser und Aceton abgetrennt und gereinigt. Durch Aufarbeitung der Mutterlaugen kann weiteres Chlorhexidin-Addukt gewonnen werden, so daß insgesamt Ausbeuten von 90 bis annähernd 100% erzielbar sind. Das gereinigte Produkt wird anschließend in bekannter Weise getrocknet und liegt danach, je nach Trocknungsgrad, in Form von Hydraten mit unterschiedlichem Wassergehalt vor.

Aufgrund seiner starken antibakteriellen Wirkung kann das erfindungsgemäße Chlorhexidin-Addukt als therapeutisches oder prophylaktisches Antiplaquemittel eingesetzt werden. Dabei verhindert es die Neubildung von Plaque und inhibiert das Wachstum von bereits vorhandenen Zahnbelägen. Erkrankungen, die durch das Vorhandensein von Plaque hervorgerufen werden, wie z.B. Parodontose, Karies und Gingivitis, sind daher mit dem erfindungsgemäßen Chlorhexidin-Addukt wirksam bekampfbar. Darüber hinaus kann es zur Desensibilisierung von empfindlichen Zahnhälsen beitragen. Bevorzugt wird es in Dentalmaterialien, wie z.B. Zahnlakken, Fissurenversieglern, Prophylaxepasten, Mundwässern, Zahnstochern, Zahnseide, Zahnkaugummi, Wundverbänden, Zahnsalben, Gingiviatrainern, Desinfektionsmitteln für Prothesen und Abformmaterialien, Trocknungsmitteln, Unterfüllungsmaterialien, Zementen, Füllungsmaterialien, Haftvermittlern und Endodontiematerialien, verwendet. Dabei kann das erfindungsgemäße Addukt auf ein festes Substrat, wie z.B. Zahnstocher oder Zahnseide, aufgebracht oder in Dentalwerkstoffe, wie z.B. provisorische Füllungsmaterialien und Fissurenversiegler, eingearbeitet werden.

Besonders vorteilhaft ist die Einarbeitung des erfindungsgemäßen Adduktes in Dentalmaterialien, die über einen beschränkten Zeitraum in der Mundhöhle verbleiben sollen, wie z.B. provisorische Füllungsmaterialien, Wundverbände, Abformmaterialien und temporäre Zemente. Wird das erfindungsgemäße Addukt beispielsweise in ein provisorisches Füllungsmaterial eingearbeit, so erhält man nach dessen Entfernen eine keimfreie Kavität, in die die endgültige Füllung unmittelbar gelegt werden kann.

Da das Chlorhexidin-Addukt nur eine recht geringe Löslichkeit in gängigen Lösungsmitteln aufweist, wird es vorzugsweise als Feststoff in die genannten Dentalmaterialien eingearbeitet. Dabei wird es in Mengen von 0,1 bis 20 Gew.%, bevorzugt 1 bis 10 Gew.% und besonders bevorzugt 3 bis 7 Gew.%, bezogen auf das gesamte Gewicht des Werkstoffes, den Dentalwerkstoffen zugesetzt. Beispiele für einsetzbare Dentalwerkstoffe sind solche, die 10 bis 95 Gew.% polymerisierbares organisches Bindemittel, 5 bis 90 Gew.% anorganische und/oder organische Füllstoffe und 0,01 bis 5 Gew.% Katalysatoren, bezogen auf das Gewicht des gesamten Werkstoffes, enthalten.

Darüber hinaus können Lösungen mit einem Gehalt von 0,03 bis 0,001 Gew% an erfindungsgemäßem Addukt verwendet werden. Als Lösungsmittel sind z.B. Wasser, Ethanol, Aceton, Ethylacetat, Triethylenglykoldimethacrylat und Decandioldimethacrylat geeignet. Ferner können künstliche oder natürliche Harze verwendet werden, die in gängigen Lösungsmitteln löslich sind und nach dem Verdunsten des Lösungsmittel hart werden. Beispiele hierfür sind Schellack, Benzoinharz, Polyvinylpyrrolidon und Kolophonium.

Eine weitere bevorzugte Anwendung des Chlorhexidin-Adduktes ist diejenige als therapeutisches oder prophylaktisches Antiplaquemittel. Dabei verhindert es die Neubildung von Zahnbelägen und inhibiert das Wachstum von bereits vorhandenen Zahnbelägen. Erkrankungen, die durch das Vorhandensein von Zahnbelägen hervorgerufen werden, wie z.B. Parodontose, primärer und sekundärer Karies und Gingivitis, lassen sich daher mit dem erfindungsgemäßen Chlorhexidin-Addukt wirksam bekämpfen.

Hinsichtlich seiner bakteriziden Wirksamkeit ist das erfindungsgemäße Addukt in einer Konzentration von 0,03 Gew.% durchaus mit dem derzeit als wirksamstes Antiplaquemittel geltenden Chlorhexidin vergleichbar. Überraschenderweise wird jedoch die Wirksamkeit von Chlorhexidin deutlich übertroffen, wenn beide in Konzentrationen von kleiner oder gleich 0,01 Gew.% eingesetzt werden. In diesem Konzentrationsbereich ist das erfindungsgemäße Chlorhexidin-Addukt auch Zinndifluorid, einer Verbindung mit bekanntermaßen sehr guten bakteriziden Eigenschaften, deutlich überlegen.

Die Überlegenheit des erfindungsgemäßen Adduktes gerade in geringen Konzentrationen ist von besonderer Bedeutung für die praktische Anwendung. Denn infolge des permanenten Speichelflusses in der Mundhöhle werden aufgebrachte Wirkstoffe laufend verdünnt. Ein Wirkstoff, wie das erfindungsgemäße Chlorhexidin-Addukt, der auch in niedrigen Konzentrationen einen starken bakteriziden Effekt zeigt, ist daher von besonderem Vorteil.

Schließlich führt der hohe Fluorgehalt des erfindungsgemäßen Adduktes dazu, daß dieses durch Fluoridierung den Zahnschmelz härten und daher auch in dieser Hinsicht wirksam vor der Bildung von Karies schützen kann. Ferner zeigt das erfindungsgemäße Chlorhexidin-Addukt den oben erwähnten Zinn-Ionen-Effekt.

Das erfindungsgemäße Addukt kann in oder auf Dentalmaterialien wie unter anderem den oben erwähnten Füllungsmassen, Zahnlacken, Fissurenversieglern, Prophylaxepasten, Zahnstochern, Zahnseide, Zahnkaugummi, Wundverbänden, Zahnsalben, Gingiviatrainern, Desinfektionsmitteln für Prothesen und Abformmaterialien, Trocknungsmitteln, Unterfüllungsmaterialien, Zementen, Füllungsmaterialien, Haftvermittlern und Endodontiematerialien, eingearbeitet oder in Form verschiedenster Zahnpflegemittel, wie z.B. Zahnpasten, Zahngele, Zahnlacke oder Mundspülungen, auf die Zähne aufgebracht werden.

Die Erfindung wird in den folgenden Beispielen näher erläutert.

### Beispiel 1

Zur Herstellung des erfindungsgemäßen Chlorhexidin-Adduktes wurden 480 ml Ethanol/Wasser (3:1) vorgelegt und 12,6 g (0,08 Mol) Zinndifluorid sowie 6 g einer 40%igen HF-Lösung (0,12 Mol) darin gelöst. Unter Rühren wurden 85 ml bzw. 90 g (0,02 Mol) einer wäßrigen 20%igen Chlorhexidindigluconat-Lösung innerhalb einer Stunde zugetropft. Nach weiteren 5 Stunden Rühren wurde der entstandene Niederschlag abfiltriert und dreimal mit 50 ml Ethanol/Wasser (3:1) gewaschen. Aus der Mutterlauge kristallisierte innerhalb einer Woche weiteres Produkt aus. Die Trocknung des erhaltenen Niederschlags erfolgte im Trockenschrank bei 50°C, die Ausbeute war nahezu quantitativ.

Das IR-Spektrum (KBr-Preßling) ist in Fig. 1 wiedergegeben.

Aus der Elementaranalyse ergibt sich, daß das Produkt Chlorhexidin-trihydrofluorid-hydrogenzinntrifluorid mit 2 Mol Kristallwasser ist.

C₂₂H₃₀N₁₀Cl₂ · 3 HF · HSnF₃ · 2 H₂O MG = 778,2

| Elementaranalyse: | | |
|---|---|---|
| | gefunden | theoretisch |
| C | 34,48% | 33,96% |
| H | 4,56% | 4,40% |
| N | 18,02% | 18,00% |
| Cl | 9,06% | 9,11% |
| F | 14,58% | 14,65% |
| Sn | 14,45% | 15,25% |
| H₂O*) | 4,84% | 4,63% |

| | | |
|---|---|---|
| *) H₂O-Gehalt bestimmt nach Karl Fischer | | |
| (Anmerkung: Der theoretische Wert für Zinn von 15,25% wird in der Elementaranalyse nicht erreicht, da bei der Synthese Chlorhexidin-Hexahydrofluorid als Nebenprodukt in einer Ausbeute von ca. 5% anfällt. Dadurch erniedrigt sich der Wert für Zinn, während für Kohlenstoff ein höherer Wert gemessen wird.) | | |

| Löslichkeit: | |
|---|---|
| Wasser | 0,03 Gew.% |
| Ethanol | 0,02 Gew.% |

### Beispiel 2

Die antibakterielle Wirksamkeit des erfindungsgemäßen Chlorhexidin-Adduktes wurde im Agar-Diffusionstest mit Streptococcus mutans nachgewiesen.

Dazu wurden Kulturabschwemmungen von Streptococcus mutans in flüssigen Hefe-Extrakt-Dextrose-Agar eingebracht. Nach Erstarren der Agarplatten wurde ein Bassin von 10 mm Durchmesser ausgestochen. Hierein wurden 0,1 ml der jeweils zu untersuchenden Lösung eingefüllt. Bei den jeweils doppelt angesetzten Proben wurden nach 24-stündiger Bebrütung bei 37°C die Durchmesser der Hemmhöfe ausgemessen. Die Ergebnisse dieser Untersuchungen sind in der nachstehenden Tabelle I wiedergegeben.

**Tabelle I**

| **Hemmhof-Durchmesser** | | | |
|---|---|---|---|
| Konzentration | Lösung A | Lösung B | Lösung C |
| 0,03 Gew.% | 17 mm | 16 mm | 20 mm |
| 0,01 Gew.% | 13 mm | 15 mm | 11 mm |
| 0,003 Gew.% | 11 mm | 11 mm | 10 mm* |

| | | | |
|---|---|---|---|
| * Keine Wirksamkeit | | | |

- Lösung A:: Wäßrige Lösung von Chlorhexidindigluconat
- Lösung B:: Wäßrige Lösung des erfindungsgemäßen Chlorhexidin-Adduktes
- Lösung C:: Wäßrige Lösung von Zinndifluorid

Es zeigt sich, daß im Konzentrationsbereich von 0,03 Gew.% die antibakterielle Wirksamkeit des erfindungsgemäßen Chlorhexidin-Adduktes gegenüber Streptococcus mutans mit der von Chlorhexidindigluconat vergleichbar ist, während Zinndifluorid in diesem Konzentrationsbereich noch eine stärkere Wirkung zeigt. Mit zunehmender Verdünnung fällt jedoch die Wirksamkeit bei den bekannten Verbindungen stark, im Falle des Zinndifluorids bei einer Konzentration von 0,03 Gew.% sogar soweit, daß kein antibakterieller Effekt mehr nachgewiesen werden kann. Demgegenüber ist die antibakterielle Wirksamkeit des erfindungsgemäßen Adduktes selbst bei Konzentrationen von 0,01 bis 0,003 Gew.% noch sehr hoch. Seine Überlegenheit gerade in niedrigen Konzentrationen macht es damit zu einem sehr wirksamen Antiplaquemittel.

### Beispiel 3

Auf die Oberfläche eines absolut planparallelen, zinnfreien Hydroxylapatit-Prüfkörpers wurde ein Dentalmaterial, wie in Beispiel 4 beschrieben, in einer Schicht von ca. 2 mm aufgebracht und 40 Sekunden mit dem Heliomat® (Lichtgerät von Vivadent) auspolymerisiert. Danach wurde der so beschichtete Prüfkörper 12 Stunden bei 37°C in destilliertem Wasser gelagert. Anschließend wurde die aufpolymerisierte Schicht unter mikroskopischer Kontrolle wieder entfernt und der Zinngehalt auf der Hydroxylapatit-Oberfläche mittels SIMS (Sekundärionen-Massenspektrometrie) analysiert. Dieses Analyseverfahren ist in Caries Res. 20, 419 (1986) beschrieben.

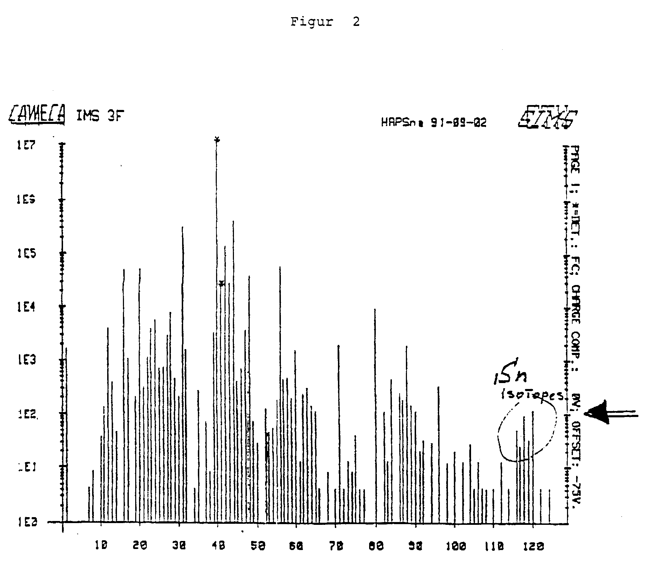

Das erhaltene Ergebnis ist in Figur 2 dargestellt, es hat sich ein beträchtlicher Teil des Zinn aus dem Dentalmaterial in der Oberfläche des Hydroxylapatits abgelagert.

### Beispiel 4

Ein lichthärtender Fissurenversiegler enthält folgende Bestandteile:
- 56,08 Gew.%: Bis-Phenol A-glycidylmethacrylat (Bis-GMA)
- 36,1 Gew.%: Triethylenglykoldimethacrylat
- 0,45 Gew.%: Cyanoethylmethylanilin
- 0,25 Gew.%: DL-Campherchinon
- 2,1 Gew.%: TiO₂
- 0,02 Gew.%: 2,6-Di-tert.-butyl-p-kresol
- 5,0 Gew.%: Chlorhexidin-Addukt

Durch Mischen aller Komponenten wurde der lichthärtbare Fissurenversiegler erhalten. Dieser wurde mit einem Pinsel auf die Fissuren eines Molaren gepinselt und 20 sec mit dem Lichthärtungsgerät Heliolux® der Firma Vivadent/Liechtenstein gehärtet. Auf diese Weise wurden die Fissuren dauerhaft verschlossen, und man erhielt durch die Fluorid-Abgabe des in dem Versiegler eingearbeiteten Chlorhexidin-Adduktes einen ausgezeichneten Kariesschutz im Okklusalbereich.

Durch die Zumischung von 1 bis 5 Gew.% des Chlorhexidin-Adduktes zu der Fissurenversiegler-Grundrezeptur wurde keine Abnahme der Durchhärtungstiefe beobachtet, wie folgende Werte für die Vikkers-Härte zeigen:

| | HV 0,5 |
|---|---|
| Fissurenversiegler ohne Chlorhexdidin-Addukt | 188 MPa |
| Fissurenversiegler + 1% Chlorhexidin-Addukt | 203 MPa |
| Fissurenversiegler + 3% Chlorhexidin-Addukt | 211 MPa |
| Fissurenversiegler + 5% Chlorhexidin-Addukt | 184 MPa |

Zum Nachweis für die Chlorhexidin- und Fluoridmigration wurden jeweils 10 Prüfkörper mit einem Durchmesser von 50 mm und einer Höhe von 0,5 mm in dest. Wasser bei 37°C gelagert. Die Fluoridionen-Konzentration wurde mittels einer Fluorelektrode bestimmt, die Chlorhexidin-Konzentration wurde UV-spektroskopisch ermittelt.

**Tabelle II**

| Kumulierte Fluorid- und Chlorhexidinabgabe | | |
|---|---|---|
| Migrationszeit [Tage] | Fluoridabgabe [µg/cm²] | Chlorhexidinabgabe [µg/cm²] |
| 1 | 1,26 | 4,30 |
| 2 | 1,97 | 5,41 |
| 3 | 2,61 | 5,96 |
| 4 | 3,13 | 6,36 |
| 7 | 4,57 | 7,11 |
| 10 | 5,77 | 8,37 |
| 17 | 8,01 | 9,07 |
| 24 | 9,83 | 9,77 |
| 30 | 11,21 | 10,37 |
| 44 | 13,87 | 10,93 |
| 86 | 18,49 | 11,01 |
| 149 | 22,91 | 11,09 |

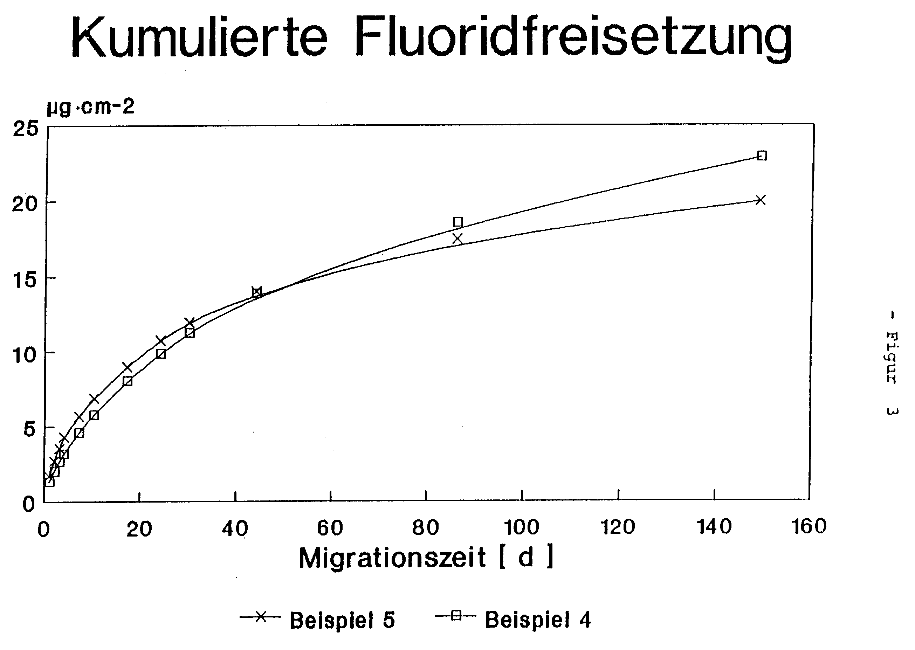

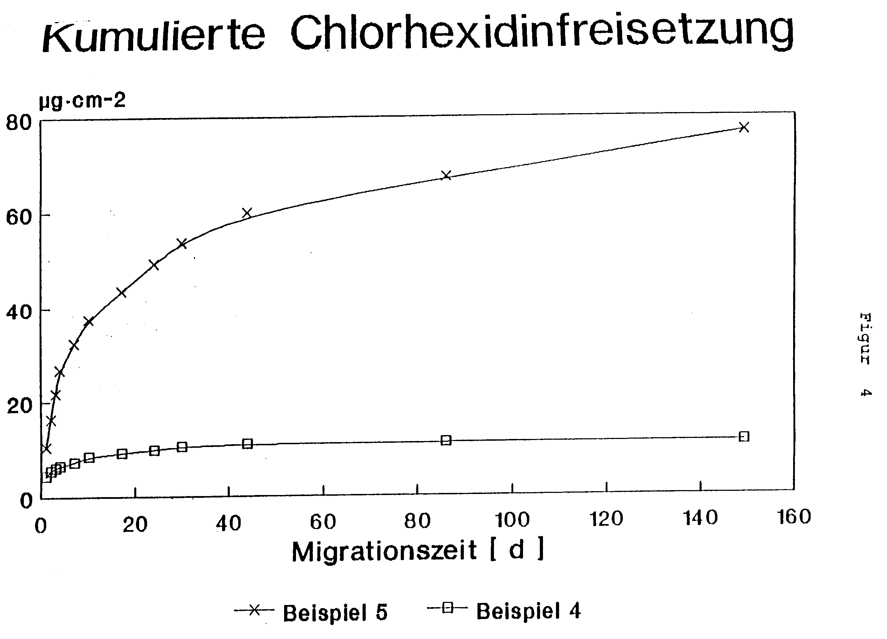

In den Figuren 3 und 4 sind die Ergebnisse graphisch dargestellt.

### Beispiel 5

Ein lichthärtender Dentalkunststoff mit relativ hoher Wasseraufnahme und somit hoher Wirkstoff-Freisetzung (z.B. geeignet als provisorisches Füllungsmaterial oder als Wundverband) hat folgende Zusammensetzung:
- 41,4 Gew.%: Polyesterurethandimethacrylat
- 0,25 Gew%: Cyanoethylmethylanilin
- 0,15 Gew.%: DL-Campherchinon
- 0,02 Gew.%: 2,6-Di.tert-butyl-p-kresol
- 33,25 Gew.%: Splitterpolymerisat
- 19,93 Gew.%: feinteiliges SiO₂ silanisiert
- 5,0 Gew.%: Chlorhexidin-Addukt

Das Splitterpolymerisat besteht aus:
- 59,4%: Urethandimethacrylat
- 40 %: feinteiliges SiO₂ silanisiert
- 0,6%: Benzpinakol.

Diese Komponenten werden zusammengemischt und bei 120°C polymerisiert. Das gefüllte Polymerisat wird zu einem Polymerpulver gemahlen. Bei dem amorphen feinteiligen silanisierten SiO₂ handelt es sich um Aerosil® OX 50 der Degussa AG.

Durch Mischen aller Komponenten wurde ein lichthärtendes Dentalmaterial erhalten.

Die Wasseraufnahme von Dentalfüllungscomposites liegt normalerweise im Bereich von 1 Gew.%, dieses Material zeigt eine Wasseraufnahme im Bereich von 3 Gew.% (3 Wochen H₂O-Lagerung bei 37°C).

Chlorhexidin- und Fluoridmigration:
Die kumulierte Fluorid- und Chlorhexidinabgabe ist in der folgenden Tabelle III zusammengefaßt.

**Tabelle III**

| Migrationszeit [Tage] | Fluoridabgabe [µg/cm²] | Chlorhexidinabgabe [µg/cm²] |
|---|---|---|
| 1 | 1,67 | 10,4 |
| 2 | 2,64 | 16,3 |
| 3 | 3,52 | 21,7 |
| 4 | 4,29 | 26,5 |
| 7 | 5,64 | 32,1 |
| 10 | 6,87 | 37,3 |
| 17 | 8,97 | 43,5 |
| 24 | 10,72 | 49,3 |
| 30 | 11,92 | 53,5 |
| 44 | 14,02 | 59,8 |
| 86 | 17,38 | 67,3 |
| 149 | 19,90 | 76,8 |

In den Figuren 3 und 4 sind die Ergebnisse graphisch dargestellt.

### Mikrobiologische Wirkung

Wie die Migrationsversuche zeigen, werden signifikante Mengen an Fluorid und Chlorhexidin aus diesem Dentalmaterial freigesetzt, so daß auch in dieser Kombination eine ausreichende Hemmung des Wachstums von Mikroorganismen zu erwarten ist.

Da nicht alle Mikroorganismen auf freigesetzte Wirkstoffe gleich reagieren, wurden Unterschungen unter Verwendung folgender Mikroben durchgeführt.
- Grampositive Bakterien:: Streptococcus mutans
Staphylococcus aureus
- Gramnegative Bakterien:: Pseudomonas auruginosa
Escherichia coli
- Pilz:: Candida albicans

Prüfkörper (d = 10 mm, h = 2 mm) wurden in die feuchten Mikroorganismen-Kulturen bei 37°C über eine Zeit von 24 Stunden eingelegt und anschließend wurde der Hemmhof bestimmt.

| | Hemmhof-Durchmesser [mm] |
|---|---|
| Streptococcus mutans | 13 |
| Staphylococcus aureus | 14 |
| Pseudomonas auruginosa | 16 |
| Escherichia coli | 14 |
| Candida albicans | 10 (keine Wirkung) |

Mit Ausnahme von Candida albicans ist eine deutliche Wachstumshemmung bei diesen unterschiedlichen Mikroorganismen feststellbar.

## Patentansprüche

1. Chlorhexidin-Addukt mit folgender Formel 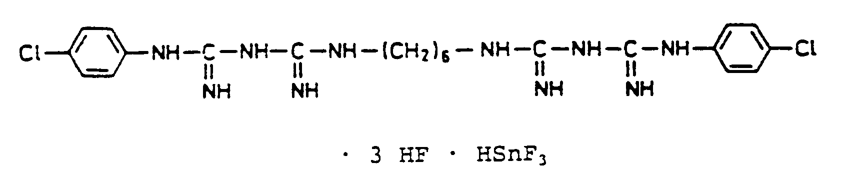 oder dessen Hydrate.

2. Verfahren zur Herstellung des Chlorhexidin-Adduktes nach Anspruch 1, dadurch gekennzeichnet, daß
a) ein Chlorhexidinsalz, Zinndifluorid und Fluorwasserstoff in einem Molverhältnis von 1 : 1 bis 4 : 4 bis 8 in einem Gemisch aus 3:1 Vol.-Teilen Ethanol/Wasser als Lösungsmittel umgesetzt werden und
b) der entstandene Niederschlag abgetrennt wird.

3. Verfahren nach Anspruch 2, dadurch gekennzeichnet, daß
a) Chlorhexidinsalz, Zinndifluorid und Fluorwasserstoff in einem Molverhältnis von 1 : 4 : 6
b) bei Raumtemperatur umgesetzt werden.

4. Verfahren nach einem der Ansprüche 2 bis 3, dadurch gekennzeichnet, daß Chlorhexidindigluconat als Chlorhexidinsalz eingesetzt wird.

5. Verwendung des Chlorhexidin-Adduktes oder seiner Hydrate nach Anspruch 1 als Antiseptikum.

6. Verwendung des Chlorhexidin-Adduktes oder seiner Hydrate nach Anspruch 1 als Mittel zur Verhütung von Karies.

7. Dentalmaterial, dadurch gekennzeichnet, daß es das Chlorhexidin-Addukt oder ein Hydrat davon gemäß Anspruch 1 enthält.

## Claims

1. A chlorhexidine adduct of the following formula 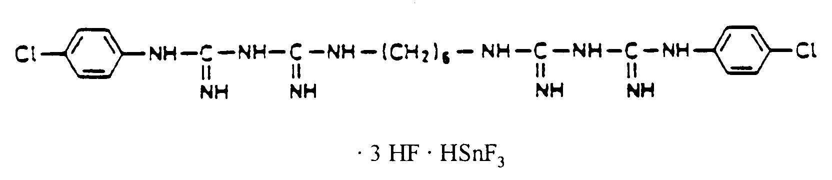 or hydrates thereof.

2. A process for producing the chlorhexidine adduct according to Claim 1, **characterized in that**
a) a chlorhexidine salt, tin difluoride and hydrofluoric acid in a molar ratio of from 1 : 1 to 4 : 4 to 8 are reacted in a mixture of 3 : 1 parts by volume of ethanol/water as a solvent, and
b) the precipitate formed is removed.

3. A process according to Claim 2, **characterized in that**
a) a chlorhexidine salt, tin difluoride and hydrofluoric acid in a molar ratio of from 1 : 4 : 6
b) are reacted at room temperature.

4. A process according to one of Claims 2 to 3 [*sic*], **characterized in that** chlorhexidine digluconate is used as the chlorhexidine salt.

5. Use of the chlorhexidine adduct or the hydrates thereof according to Claim 1 as an antiseptic.

6. Use of the chlorhexidine adduct or the hydrates thereof according to Claim 1 as a caries-preventing agent.

7. A dental material, **characterized in that** it contains the chlorhexidine adduct or a hydrate thereof according to Claim 1.

## Revendications

1. Produit d'addition de la chlorhexidine de formule suivante 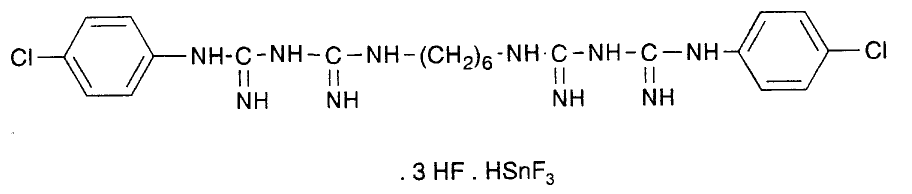 ou ses hydrates.

2. Procédé pour la préparation du produit d'addition de la chlorhexidine selon la revendication 1 caractérisé en ce que
a) on fait réagir un sel de chlorhexidine, du difluorure d'étain et du fluorure d'hydrogène en proportions molaires de 1 : 1 à 4 : 4 à 8 dans un mélange éthanol/eau de 3:1 parties volumiques comme solvant et
b) on sépare le précipité obtenu.

3. Procédé selon la revendication 2, caractérisé en ce que
a) on fait réagir un sel de chlorhexidine, du difluorure d'étain et du fluorure d'hydrogène dans les proportions molaires de 1 : 4 : 6
b) à température ambiante.

4. Procédé selon l'une des revendications 2 ou 3, caractérisé en ce que l'on utilise le digluconate de chlorhexidine comme sel de chlorhexidine.

5. Utilisation du produit d'addition de la chlorhexidine ou de ses hydrates selon la revendication 1 comme antiseptique.

6. Utilisation du produit d'addition de la chlorhexidine ou de ses hydrates selon la revendication 1 comme agent pour la prévention des caries.

7. Matériau dentaire caractérisé en ce qu'il contient le produit d'addition de la chlorhexidine ou un de ses hydrates selon la revendication 1.
